# EUROPÄISCHE PATENTSCHRIFT

(11) **EP 1 621 163 B1**
(45) Veröffentlichungstag und Bekanntmachung des Hinweises auf die Patenterteilung: **12.09.2012**
(21) Anmeldenummer: 05013596.1
(22) Anmeldetag: 23.06.2005
(51) Int. Cl.: A61F 2/46

(54) **Chirurgisches Instrument zum Einführen einer mehrteiligen Zwischenwirbelprothese**
Surgical instrument for inserting multipart intervertebral implants
Instrument chirurgical pour l'introduction d'implants intervertébraux en plusieurs parties

(30) Priorität: 28.07.2004 US 900647
(43) Veröffentlichungstag der Anmeldung: 01.02.2006
(73) Patentinhaber: Weber Instrumente GmbH, 78576 Emmingen-Liptingen (DE)
(72) Erfinder: Weber, Helmut, 78576 Emmingen-Liptingen (DE)
(74) Vertreter: Patentanwälte Westphal, Mussgnug & Partner

(56) Entgegenhaltungen:
- EP-A- 1 222 903
- DE-U1- 20 310 433
- US-A- 5 314 477
- US-A1- 2004 117 022

## Beschreibung

Die Erfindung betrifft ein chirurgisches Instrument zum Einführen einer mehrteiligen Zwischenwirbelprothese.

Zum Einsetzen von mehrteiligen, insbesondere dreiteiligen Zwischenwirbelprothesen, die aus zwei Prothesenplatten, welche jeweils mit einem Wirbelkörper verbunden werden, und einem dazwischen angeordneten Prothesenkern bestehen, sind verschiedene Instrumente bekannt.

Aus der EP 0 333 990 A2 ist ein chirurgisches Instrument zum Einsetzen von Zwischenwirbelprothesen bekannt, welches aus einer Spreizzange besteht, die an ihrem vorderen Ende an jeder Spreizbacke eine Halterung für eine Prothesenplatte aufweist. Die beiden Prothesenplatten können zunächst mit der Spreizzange stark angenähert werden, um sie in den Zwischenraum zwischen den angrenzenden Wirbeln einzubringen. Anschließend wird die Spreizzange aufgespreizt, um den Prothesenkern zwischen die Prothesenplatten einführen zu können.

Die DE 299 19 078 U1 offenbart ein chirurgisches Instrument zum Einsetzen von Zwischenwirbelimplantaten, welches zwei Führungsschienen aufweist, die am hinteren Ende schwenkbar gelagert sind und an ihrem freien Ende je eine Halteeinrichtung für eine Prothesenplatte aufweisen. Zwischen den beiden Armen ist eine Längsführung für den Prothesenkern angeordnet. Zunächst werden die beiden Prothesenplatten in angenähertem Zustand in den Zwischenwirbelraum eingeführt, um anschließend die beiden Prothesenhalterungen auseinander zu spreizen, wobei gleichzeitig der Prothesenkern zum distalen Ende des Instruments geschoben wird, bis dieser die gewünschte Endstellung zwischen den Prothesenplatten erreicht.

Die DE 102 25 703 A1 offenbart ein Instrument zum Einführen einer Zwischenwirbelprothese, welches zwei durch eine Parallelführung verbundene, voneinander wegspreizbare Prothesenhalterungen für ein Paar von Prothesenplatten aufweist, wobei sämtliche, die beiden Prothesenhalterungen verbindenden Teile außerhalb einer mittleren, in Längsrichtung des Instruments verlaufenden Durchtrittsöffnung angeordnet sind, deren Weite mindestens den Querabmessungen des zwischen die Prothesenplatten einzusetzenden Prothesenkerns und eines dafür vorgesehenen Prothesenkernhalters entspricht. Wiederum werden zunächst die beiden Prothesenplatten mit Hilfe des Instruments eingesetzt, um anschließend die Prothesenplatten auseinander zu spreizen und den Prothesenkern einzusetzen.

Die Prothesenplatten weisen auf ihren zueinander zugewandten Flächen konkave Ausnehmungen auf, zwischen die der Prothesenkern gesetzt wird, der ähnlich geformte konvexe Vorsprünge aufweist. Die gewölbten Flächen der Prothesenplatten und des Prothesenkerns können in gewissen Grenzen aufeinander gleiten und ermöglichen so ein Verkippen und Verdrehen der oberen Prothesenplatte gegenüber der unteren, wodurch die Beweglichkeit des Wirbelsäulenabschnitts, in welchen die Zwischenwirbelprothese eingesetzt wird, gewährleistet ist.

Um den Prothesenkern zwischen die beiden Prothesenplatten einsetzen zu können, müssen daher die Prothesenplatten verhältnismäßig weit auseinander gespreizt werden, damit die konvexen Vorsprünge des Prothesenkerns über den Rand der konkaven Vertiefungen in den Prothesenplatten zwischen die Prothesenplatten rutschen können. Eine derartige große Aufspreizung des Zwischenwirbelraums soll jedoch nach Möglichkeit vermieden werden, um Beschädigungen der Wirbelsäule und der Wirbelkörper ausschließen zu können. Weiterhin wird der Prothesenkern gewöhnlich derart eingesetzt, dass der Zwischenwirbelraum nicht vollständig aufgespreizt wird, um den Prothesenkern ohne Kraftaufwand einsetzen zu können, sondern dass der Prothesenkern in einen kleineren Zwischenraum zwischen den Prothesenplatten eingeschlagen wird, wobei nur in dem Augenblick, in welchem die konvexen Vorsprünge des Prothesenkerns über den Rand der konkaven Vertiefungen in den Prothesenplatten rutschen, der Zwischenwirbelraum besonders weit aufgespreizt wird. Der Prothesenkern ist dabei jedoch großen Belastungen ausgesetzt, da über ihn die Kraft einwirkt, welche den Zwischenwirbelraum aufspreizt, sodass die Oberfläche des Prothesenkerns beschädigt werden kann.

Als weiterer Stand der Technik werden die EP 1 222 903 A1, die DE 203 10 433 U1, die US 5,314,477 und die EP 0 333 990 genannt.

Die Aufgabe der Erfindung besteht daher darin, ein chirurgisches Instrument zum Einsetzen von Zwischenwirbelprothesen anzugeben, welches leichter handhabbar ist und gleichzeitig ein möglichst schonendes Einsetzen der Zwischenwirbelprothese sowohl für den Patienten als auch für den Prothesenkern ermöglicht.

Die Aufgabe wird erfindungsgemäß gelöst durch ein chirurgisches Instrument gemäß dem Patentanspruch 1.

Vorteilhafte Ausgestaltungen und Weiterbildungen der Erfindung sind in den Unteransprüchen angegeben.

Das chirurgische Instrument zum Einführen einer wenigstens dreiteiligen Zwischenwirbelprothese gemäß der Erfindung weist am distalen Ende eines Griffelements eine U-förmige Halterung auf, in welcher die Zwischenwirbelprothese bestehend aus zwei Prothesenplatten und einem Prothesenkern mit sämtlichen Bestandteilen lösbar einsetzbar ist, wobei die einzelnen Bestandteile zueinander fixiert ausgerichtet sind.

Mit dem erfindungsgemäßen chirurgischen Instrument wird die Zwischenwirbelprothese mit sämtlichen Bestandteilen gleichzeitig in den Zwischenwirbelraum eingeführt, welcher dabei mittels eines weiteren Instruments zum Aufspreizen geöffnet gehalten wird. Der Vorteil liegt zunächst darin, dass der Prothesenkern bereits zwischen die beiden Prothesenplatten eingebracht ist, so dass kein nachträgliches Einbringen des Prothesenkerns zwischen die Prothesenplatten und somit kein vergrö-βertes Aufspreizen des Zwischenwirbelraums vonnöten ist. Ein weiterer Vorteil liegt darin, dass sämtliche Bestandteile der Zwischenwirbelprothese zueinander ausgerichtet gehalten sind, so dass gewährleistet ist, dass auch im in den Zwischenwirbelraum eingesetzten Zustand kein Verkanten oder Verdrehen der Prothesenplatten gegeneinander möglich ist. Die U-förmige Halterung des erfindungsgemäßen Instruments gewährleistet, dass die Zwischenwirbelprothese von der Zugangsseite aus sicher zwischen die Wirbel eingesetzt und anschließend wieder entfernt werden kann, ohne die Zwischenwirbelprothese anschlie-βend zu verdrehen oder sonst unnötig zu bewegen.

Im Anschluss an die U-förmige Halterung ist ein entlang der Achse des Griffelements bewegbarer Anschlag angeordnet. Dieser Anschlag dient dazu zu definieren, wann die Zwischenwirbelprothese die gewünschte Endposition beim Einführen in den Zwischenwirbelraum erreicht hat. In der Regel wird der Zwischenwirbelraum, welcher bei der Operation nach Entfernung der defekten Bandscheibe entsteht, mit einer Kontrollprothese sondiert, um die bevorzugten Ausmaße der Zwischenwirbelprothese und die genaue Positionierung derselben zu sondieren. Um entsprechend an dem erfindungsgemäßen chirurgischen Instrument zum Einführen der Zwischenwirbelprothese sehen zu können, wie weit das Instrument mit der Zwischenwirbelprothese in den Zwischenwirbelraum eingeführt werden muss, um die Zwischenwirbelprothese optimal zu positionieren, kann der mit der Kontrollprothese bestimmte Wert an dem bewegbaren Anschlag eingestellt werden. Sobald der Anschlag auf die Außenkontur des angrenzenden Wirbels schlägt, ist die optimale Position für die Zwischenwirbelprothese erreicht.

Vorzugsweise weist die U-förmige Halterung Klemmelemente für die Zwischenwirbelprothese auf. In diesem Fall kann die Zwischenwirbelprothese mit dem chirurgischen Instrument in den Zwischenwirbelraum eingeführt werden, woraufhin die Aufspreizung des Zwischenwirbelraums zurückgenommen wird. Die auf der Außenseite der Prothesenplatten angeordneten Zähne greifen dabei in die angrenzenden Wirbel ein. Dieser Eingriff genügt, um beim Zurückführen des chirurgischen Instruments die Klemmung der Zwischenwirbelprothese in der U-förmigen Halterung zu überwinden, so dass kein zusätzlicher Mechanismus zum Lösen der Prothese vonnöten ist.

Bei einer vorteilhaften Weiterbildung der Erfindung sind die Klemmelemente als zwei auf der Innenseite entlang der Schenkel der U-förmigen Halterung verlaufende Blattfedern ausgebildet. Die Blattfedern lassen sich besonders einfach fertigen und können zusätzlich einstückig an die U-förmige Halterung angeformt sein.

Vorzugsweise halten die Klemmelemente den Prothesenkern. Insbesondere in Kombination mit einer Ausrichtevorrichtung für die Prothesenplatten genügt dies, um die Zwischenwirbelprothese sicher zu fixieren.

Bei einer vorteilhaften Weiterbildung der Erfindung ist die Innenkontur der U-förmigen Halterung korrespondierend zu der Außenkontur des Prothesenkerns ausgebildet. Auf diese Weise wird gewährleistet, dass der Prothesenkern besonders sicher gehalten wird.

Vorzugsweise ist die Innenkontur der U-förmigen Halterung als Kreisbogen von etwa 200° ausgebildet. Die Innenkontur der U-förmigen Halterung umfasst somit geringfügig mehr als die Hälfte des kreisförmigen Prothesenkerns und weist zwischen den Schenkeln der U-förmigen Halterung eine Öffnung auf, welche geringer als der Durchmesser des Prothesenkerns ist. Auf diese Weise wird zuverlässig eine Klemmung des Prothesenkerns erreicht.

Bei einer vorteilhaften Weiterbildung der Erfindung sind in der Ebene der U-förmigen Halterung auf der Innenseite der Schenkel der U-förmigen Halterung je zwei Führungsschienen zur Aufnahme der Prothesenplatten ausgeformt. Die Prothesenplatten können somit als Einzelelement ohne Wiederstand in die Führungsschienen geschoben werden. Eine Halterung der Zwischenwirbelprothese wird dadurch erreicht, dass die konvexen Vorsprünge des Prothesenkerns in die konkaven Ausnehmungen der Prothesenplatten eingreifen und somit auch die Prothesenplatten sicher in der U-förmigen Halterung gehalten werden, obwohl keine Klemmung der Prothesenplatten vorliegt. Weiterhin gewährleisten die Führungsschienen zur Aufnahme der Prothesenplatten eine Ausrichtung der Prothesenplatten in Bezug auf den Prothesenkern und zueinander. Dazu sind vorzugsweise die Führungsschienen zur Aufnahme der Prothesenplatten parallel zu einander angeordnet. Vorzugsweise sind dazu die Führungsschienen weiterhin beidseitig der entlang der Schenkel der U-förmigen Halterung verlaufenden Plattfedern angeordnet..

Der Anschlag ist vorzugsweise senkrecht zur Ebene der U-förmigen Halterung angeordnet, um einen definierten Anschlagpunkt zu gewährleisten.

Vorzugsweise durchsetzt der Anschlag das Griffelement senkrecht und ragt beidseitig des Griffelements hervor. Auf diese Weise sichergestellt, dass der bedienenden Person der Blick auf den Anschlag möglich ist, unabhängig davon, ob das chirurgische Instrument um 180° um die Längsachse gedreht verwendet wird.

Vorzugsweise greift in den Anschlag ein Gewinde ein, welches entlang der Achse des Griffelements verläuft und mittels eines Schraubelements verstellbar ist. Dies ermöglicht eine einfache Bewegung des Anschlags entlang der Längsachse des Griffelements und stellt sicher, dass der Anschlag beim Auftreffen auf den Wirbelkörper nicht durch Druck auf den Anschlag entlang einer Achse parallel zur Längsachse des Griffelements verstellt werden kann.

Bei einer vorteilhaften Weiterbildung der Erfindung ist die U-förmige Halterung zweiteilig ausgebildet, wobei die beiden Halterungselemente der U-förmigen Halterung über eine Parallelführung verbunden und aufspreizbar sowie wieder schließbar sind. Eine derartige Ausgestaltung der U-förmigen Halterung ermöglich es, Zwischenwirbelprothesen unterschiedlicher Höhe mit ein und dem selben Instrument halten zu können. Somit werden für unterschiedliche Zwischenwirbelprothesen, die vonnöten sind, um die unterschiedlichen Ausgestaltungen der Bandscheiben verschiedener Personen gerecht zu werden, für die unterschiedlichen Höhen der Zwischenwirbelprothesen keine verschiedenen chirurgischen Instrumente benötigt.

Vorzugsweise weisen die beiden Elemente der U-förmigen Halterung an ihrer einander abgewandten Seite auf der Innenseite der Schenkel je eine Führungsschiene zur Aufnahme der Prothesenplatte auf. Da in der Regel die Höhe der Zwischenwirbelprothese durch die Höhe des Prothesenkerns variiert wird, ist damit sichergestellt, dass der Zwischenraum zwischen den eingespannten Prothesenplatten in der U-förmigen Halterung variiert werden kann.

Die Erfindung wird anhand der folgenden Figuren ausführlich erläutert. Es zeigt
- Figur 1a: eine Seitenansicht einer dreiteiligen Zwischenwirbelprothese,
- Figur 1b: einen Axialschnitt durch die Zwischenwirbelprothese gemäß Figur 1a,
- Figur 1c: eine Aufsicht auf die Zwischenwirbelprothese gemäß Figur 1a,
- Figur 1d: einen Axialschnitt durch ein zweites Ausführungsbeispiel einer Zwischenwirbelprothese,
- Figur 2a: eine Aufsicht auf ein erstes Ausführungsbeispiel eines chirurgischen Instruments zum Einführen einer Zwischenwirbelprothese,
- Figur 2b: eine Ansicht von oben axial auf das Ausführungsbeispiel gemäß Figur 1d,
- Figur 3a: eine Draufsicht auf ein zweites Ausführungsbeispiel eines chirurgischen Instruments zum Einführen einer Zwischenwirbelprothese,
- Figur 3b: eine Draufsicht auf das Ausführungsbeispiel gemäß Figur 3a mit zurückgestelltem Anschlag,
- Figur 3c: eine Ansicht von oben axial auf das Ausführungsbeispiel gemäß Figur 3a,
- Figur 3d.: eine Seitenansicht auf das Ausführungsbeispiel gemäß Figur 3a,
- Figur 3f: einen Axialschnitt durch das Ausführungsbeispiel gemäß Figur 3a im zusammengeführten Zustand,

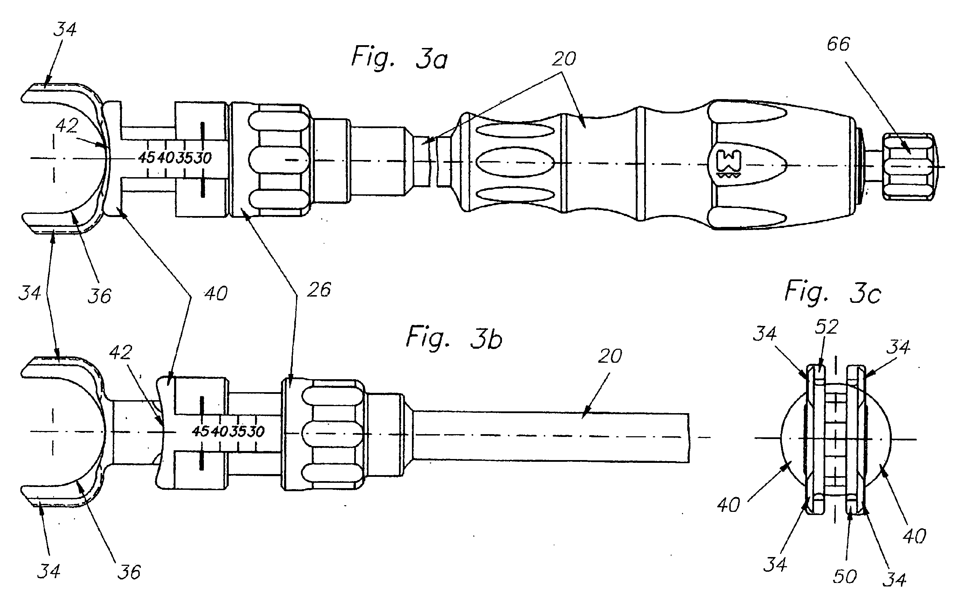

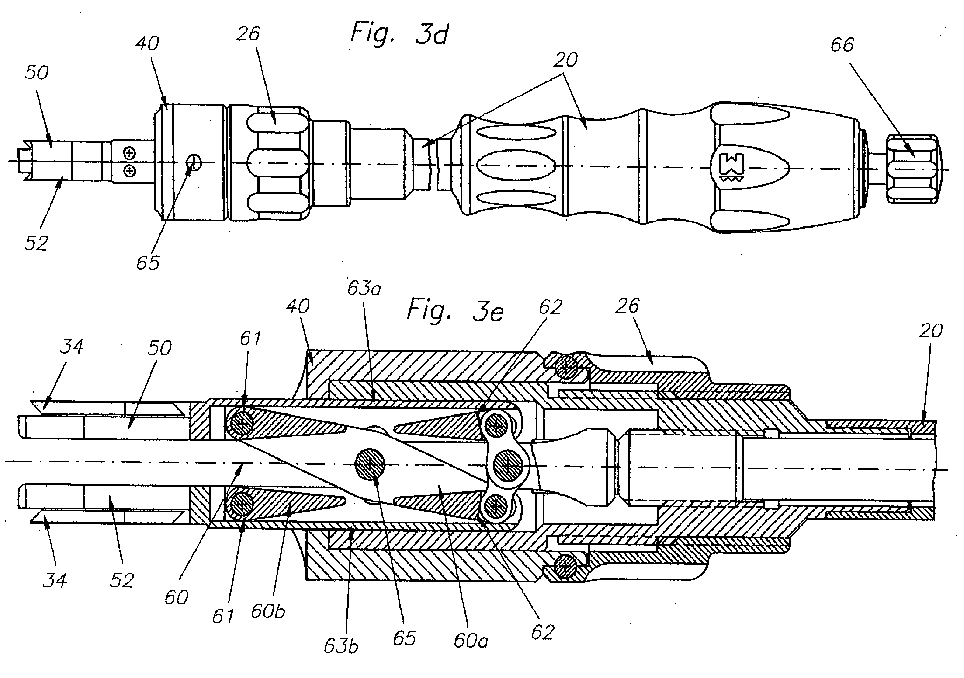

- Figur 3e: einen Axialschnitt durch das Ausführungsbeispiel gemäß Figur 3a im aufgespreizten Zustand.

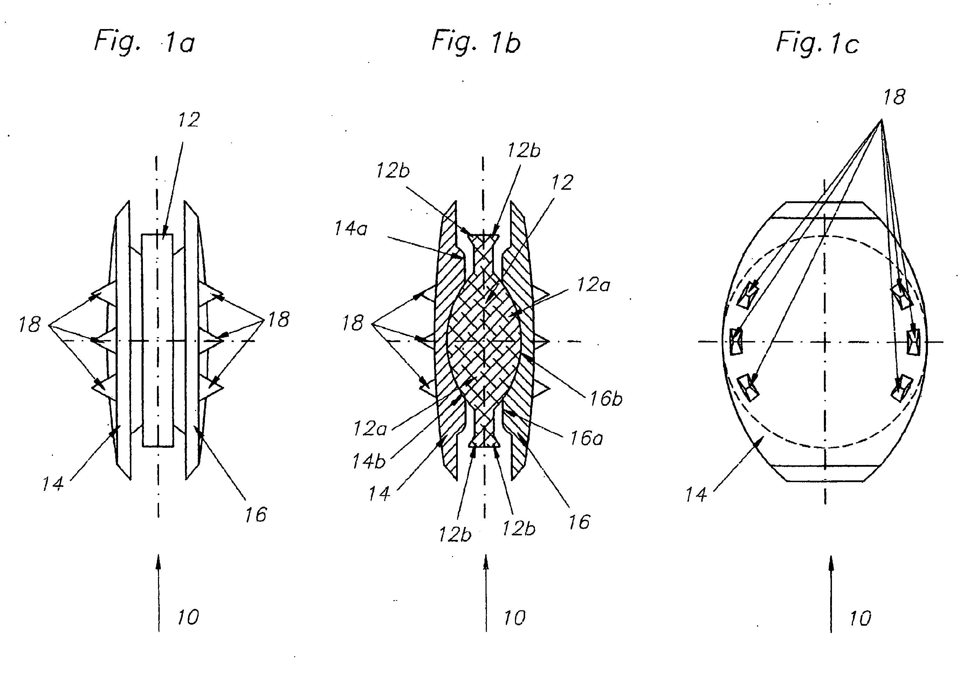

Figuren 1a, 1b und 1c zeigen eine Zwischenwirbelprothese 10 bestehend aus einem Prothesenkern 12 und zwei Prothesenplatten 14, 16, welche den Prothesenkern 12 einschließen. Der Prothesenkern 12 besteht aus einer kreisförmigen Scheibe, welche beidseitig jeweils ein axial angeordnetes Kugelsegment 12a und einen am äußeren Rand umlaufenden axial vorspringenden Ringsteg 12b aufweist.

Die beiden Prothesenplatten 14 und 16 sind identisch ausgebildet und bestehen aus einer ellipsenförmigen Scheibe, deren Hauptscheitel abgeflacht sind, wobei auf einer Seite der Scheibe ein kreisförmiger Ringwulst 14a, 16a angeordnet ist, welcher eine kugelschalenförmige Vertiefung 14b, 16b aufweist. In zusammengesetztem Zustand der Zwischenwirbelprothese 10 liegen die Kugelsegmente 12a des Prothesenkerns 12 in den beiden kugelschalenförmigen Vertiefungen 14b, 16b der Prothesenplatten 14, 16. Auf der dem Prothesenkern 12 abgewandten Seite der Prothesenplatten 14, 16 sind mehrere, vorliegend sechs Zähne 18 angeordnet, welche die Prothesenplatten 14, 16 in den angrenzenden Wirbeln verankern. In dem vorliegenden Ausführungsbeispiel sind die dem Prothesenkern 10 abgewandten Seiten der Prothesenplatten 14, 16 eben ausgebildet. Um die Prothesenplatten 14, 16 an Neigungen der angrenzenden Wirbelkörper anpassen zu können, kann die dem Prothesenkern 10 abgewandte Seite der Prothesenplatten 14, 16 gegen die Ebene der Prothesenplatte 14, 16 geneigt sein.

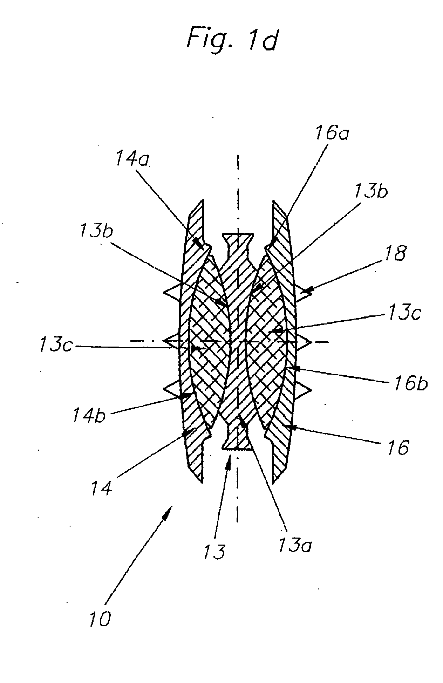

Figur 1d zeigt einen Axialschnitt durch eine weitere Ausführungsform der Zwischenwirbelprothese 10, bestehend aus den beiden Prothesenplatten 14, 16 und einem dreiteiligen Prothesenkern 13. Der Prothesenkern 13 ist dabei so ausgebildet, dass in ein scheibenförmiges Mittelelement 13a beidseitig je eine kugelschalenförmige Vertiefung 13b eingeformt ist, in welchen je ein aus je zwei Kugelsegmenten gleichen Durchmessers gebildetes Gleitelement 13c eingesetzt ist. Das dem Mittelelement 13a zugewandte Kugelsegment der Gleitelemente 13c ist somit in den kugelschalenförmigen Vertiefungen 13b des Mittelelements 13a gleitend angeordnet, während das dem Mittelelement 13a abgewandte Kugelsegment der Gleitelemente 13c in den kugelschalenförmigen Vertiefungen 14b, 16b der Prothesenplatten 14, 16 zu liegen kommen und in diesen gleitend angeordnet sind. Ein mehrteilig ausgebildeter Prothesenkern 13 ermöglicht weitere Freiheitsgrade der Bewegung der beiden Prothesenplatten 14, 16 gegeneinander, wodurch die Beweglichkeit des Wirbelsäulenabschnitts, in welchen die Zwischenwirbelprothese 10 implantiert wurde, weiter erhöht wird.

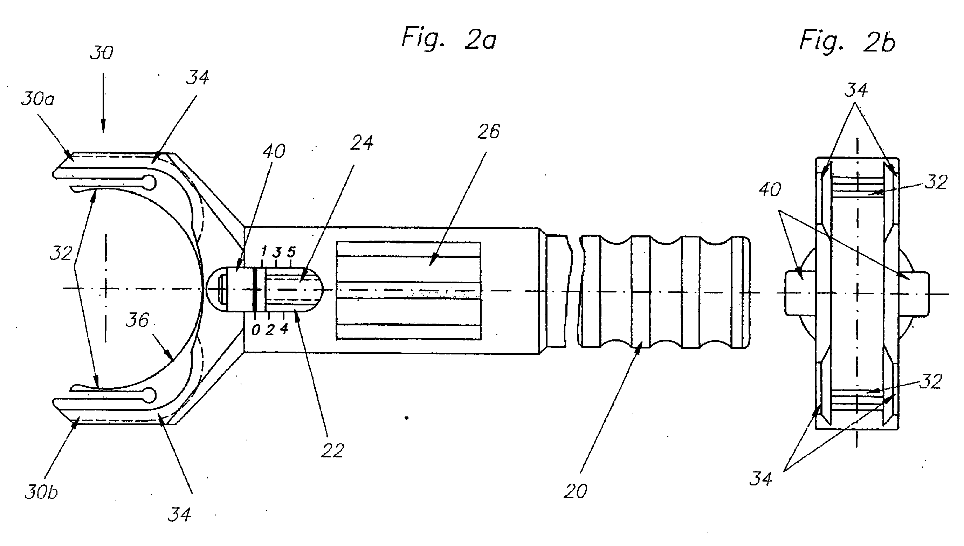

Figur 2a und 2b zeigen zwei Ansichten eines ersten Ausführungsbeispiels eines chirurgischen Instruments zum Einführen einer mehrteiligen Zwischenwirbelprothese 10, bestehend aus einem Griffelement 20, an dessen distales Ende sich eine U-förmige Halterung 30 anschließt. Dabei liegt die Achse des Griffelements 20 in der durch die U-förmige Halterung 30 aufgespannten Ebene. Die U-förmige Halterung 30 weist zwei Schenkel 30a, 30b auf, an deren Innenseite parallel zu den Schenkel 30a, 30b jeweils eine Blattfeder 32 angeordnet ist. Die Innenkontur 36 der U-förmigen Halterung 30, gebildet im Wesentlichen durch die beiden Blattfedern 32, ist als Kreisbogen von etwa 200° ausgebildet. Auf diese Weise ist die Öffnung zwischen den distalen Enden der Blattfedern 32 geringfügig kleiner als der maximale Durchmesser des Kreisbogens. Wird somit der scheibenförmige Prothesenkern 12 mit seiner kreisfömigen Außenkontur zwischen die beiden Blattfedern 32 eingebracht, geben die Blattfedern 32 zunächst geringfügig nach und legen sich anschließend an die Außenkontur des Prothesenkerns 12 an. Auf diese Weise ist eine sichere Klemmung des Prothesenkerns 12 zwischen den Blattfedern 32 gewährleistet.

Auf der Innenseite der Schenkel 30a, 30b der U-förmigen Halterung 30 sind beidseitig entlang der Blattfedern 32 Führungsschienen 34 zur Aufnahme der Prothesenplatten 14, 16 eingelassen. Der Abstand der beiden Schenkel 30a, 30b entspricht dabei der Länge der Hauptachse der im Wesentlichen ellipsenförmigen Scheibe der Prothesenplatten 14, 16. Die Führungsschienen 34 verlaufen auf jedem der Schenkel 30a, 30b parallel zueinander, um eine parallele Ausrichtung der Prothesenplatten 14,16 zu gewährleisten. Der Abstand der Führungsschienen 34 auf jedem der Schenkel 30a, 30b entspricht dabei dem Abstand der Prothesenplatten 14,16 im zusammengesetzten Zustand der Zwischenwirbelprothese 10. Wird die Zwischenwirbelprothese 10 in die U-förmige Halterung 30 eingesetzt, wird nur der Prothesenkern 12 über die Klemmung gehalten. Die Prothesenplatten 14, 16 lassen sich prinzipiell ohne Wiederstand in den Führungsschienen bewegen. Sie werden jedoch aufgrund des Eingriffs der Kugelsegmente 12a des Prothesenkerns 12 in die kugelschalenförmigen Vertiefungen 14b, 16b und die exakt parallele Ausrichtung der Prothesenplatten 14, 16 zueinander ebenfalls sicher in der U-förmigen Halterung 30 gehalten.

Im Anschluss an die U-förmige Halterung 30 ist in dem Griffelement 20 ein entlang der Achse des Griffelements 20 bewegbarer Anschlag 40 angeordnet. Der Anschlag 40 durchsetzt das Griffelement in einer radialen Öffnung 22 und ragt beidseitig des Griffelements 20 hervor. Dabei steht der Anschlag senkrecht zur Achse des Griffelements und senkrecht zur Ebene der U-förmigen Halterung 30. Der Anschlag 40 wird von einem entlang der Achse des Griffelements 20 verlaufenden Gewinde 24 durchsetzt, welches mittels eines Schraubelements 26 verdrehbar ist. Da der Anschlag 40 unverdrehbar in der radialen Öffnung 22 eingepasst ist, bewegt sich der Anschlag 40 entlang der Achse des Griffelements 20, sobald das Schraubelement 26 und damit das Gewinde 24 gedreht werden. Wird der Anschlag 40 weitmöglichst in Richtung des distalen Endes des chirurgischen Instruments bewegt, wird die Zwischenwirbelprothese 10 weniger weit in den Zwischenwirbelraum eingeführt als wenn der Anschlag 40 soweit wie möglich vom distalen Ende des chirurgischen Instruments entfernt wird und somit beim Einführen des Instruments in den Zwischenwirbelraum erst später auf die benachbarten Wirbel auftrifft.

In Figur 2b ist eine Ansicht von oben axial auf das erste Ausführungsbeispiel gezeigt, in welcher insbesondere die parallel verlaufenden Führungsschienen 34 zur Aufnahme der Prothesenplatten 14, 16 deutlich zu erkennen sind. Weiterhin zeigt Figur 2b den Zwischenraum zwischen den Blattfedern 32 und den Führungsschienen 34, in welchen der Prothesenkern 12 einsetzbar ist und in welchem dieser durch die Blattfedern 32 lösbar fixiert ist.

Die Figuren 3a bis 3f zeigen ein zweites Ausführungsbeispiel eines chirurgischen Instruments zum Einführen der Zwischenwirbelprothese 10. Das zweite Ausführungsbeispiel des erfindungsgemäßen chirurgischen Instruments weist eine U-förmige Halterung bestehend aus zwei Halterungselementen 50, 52 auf. Jedes der Elemente 50, 52 ist im Wesentlichen identisch zu der U-förmigen Halterung 30, wobei jedoch in jedem der Halterungselemente 50, 52 nur eine Führungsschiene 34 angeordnet ist. Die beiden Halterungselemente 50, 52 sind derart an dem distalen Ende des Griffselements 20 angeordnet, dass die Seiten der Halterungselemente 50, 52, welche die Führungsschiene 34 aufweisen, auf voneinander abgewandten Seiten liegen, während die Seiten der Halterungselemente 50, 52, welche keine Führungsschiene aufweisen, einander zugewandt sind. Liegen die beiden Halterungselemente 50, 52 direkt aufeinander, wie in Figur 3d gezeigt, ergibt sich im Wesentlichen die U-förmige Halterung 30, welche in der durch das U aufgespannten Ebene symmetrisch geteilt wurde.

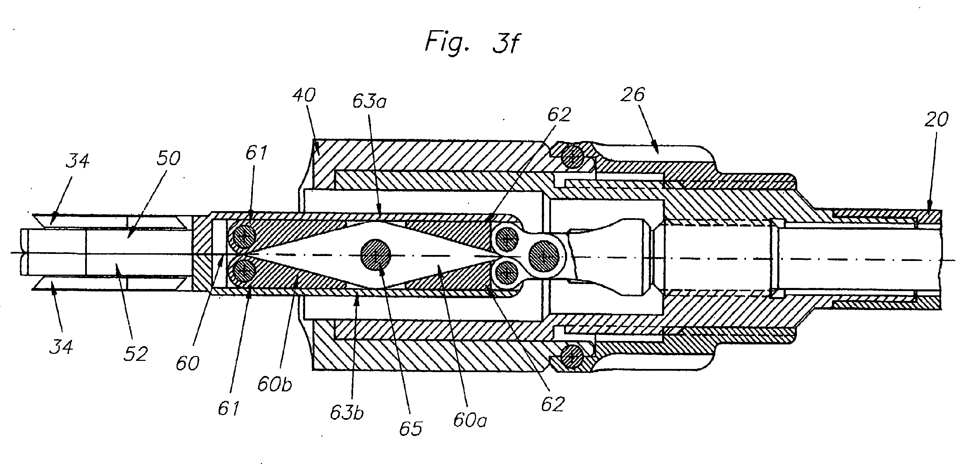

Die beiden Halterungselemente 50, 52 sind über eine Parallelführung 60 verbunden und aufspreizbar. Die Figuren 3e und 3f zeigen je einen Axialschnitt durch das zweite Ausführungsbeispiel des erfindungsgemäßen Instruments mit zwei unterschiedlichen Positionen der Parallelführung 60.

Die Parallelführung 60 ist in an sich bekannter Art nach Art einer Schere mit zwei Scherengliedern 60a, 60b ausgebildet, welche in ihrer Symmetrieachse über eine Achse 65 gegeneinander verdrehbar miteinander verbunden sind. An den auf der zum distalen Ende des Instruments weisenden Seite der Achse 65 liegenden freien Enden 61 der Scherenglieder 60a, 60b sind die Halterungselemente 50, 52 angeordnet. Die auf der anderen Seite der Achse gelegenen freien Enden 62 der Scherenglieder 60a, 60b stehen mit einer in an sich bekannter Weise ausgestalteten Vorrichtung in Eingriff, welche diese freien Enden 62 aufeinander zu und voneinander weg bewegen kann. Diese Vorrichtung kann über einen Drehknopf 66, welcher am proximalen Ende des Instruments angeordnet ist, betätigt werden. Die Anordnung des Drehknopfs 66 am proximalen Ende des Instruments ist von Vorteil, weil er sich somit außerhalb des Operationsbereichs befindet und somit auch im in den Operationsbereich eingebrachten Zustand des distalen Endes des Instruments leicht betätigen lässt.

Zusätzlich ist jeweils das freie Ende 61 des Scherengliedes 60a, 60b mit dem freien Ende 62 des anderen Scherengliedes 60b, 60a über ein Verbindungselement 63a, 63b verbunden, in deren Verlängerung zum distalen Ende des Instruments über die freien Enden 61 der Scherenglieder 60a, 60b hinaus die Halterungselemente 50, 52 angeordnet sind. Werden die freien Enden 62 voneinander wegbewegt, werden die Halterungselemente 50, 52, welche an den gegenüberliegenden freien Enden 61 der Scherenglieder 60a, 60b drehbar gelagert angeordnet sind, parallel voneinander wegbewegt. Durch eine derartige Ausgestaltung der U-förmigen Halterung 30 wird ermöglicht, dass Zwischenwirbelprothesen 10 unterschiedlicher Dicken mit ein und demselben Instrument in den Zwischenwirbelraum eingeführt werden können.

Eine Vielzahl von chirurgischen Instrumenten für unterschiedliche Höhen der Zwischenwirbelprothesen 10 erübrigt sich somit.

In dieser Ausführungsform des Instruments sind keine Klemmelemente, welche parallel zu den Schenkeln der U-förmigen Halterung verlaufen und den Prothesenkern entlang seiner Außenkontur klemmen, vonnöten. Die Zwischenwirbelprothese wird ausreichend darüber gehalten, dass die beiden Prothesenplatten 14, 16 in die Führungsschienen 34 in jeweils einem Halterungselement 50, 52 eingesetzt werden, der Prothesenkern 12 dazwischen gesetzt und durch Zusammenführen der Halterungselemente 50, 52 klemmend fixiert wird.

Die Figuren 3a und 3b zeigen das zweite Ausführungsbeispiel in einer Draufsicht mit zwei unterschiedlichen Positionierungen des Anschlags 40. Das zweite Ausführungsbeispiel unterscheidet sich von dem ersten Ausführungsbeispiel auch in der Ausgestaltung des Anschlags 40, da die Mechanik zum Verstellen des Anschlags 40 nicht innerhalb des Griffelements 20, sondern außerhalb des Griffelements 20 angeordnet ist, da innerhalb des Griffelements 20 die Mechanik der Parallelführung 60 angeordnet ist.

Der Anschlag 40 des zweiten Ausführungsbeispiels weist eine Anlagekontur 42 auf, welche dem Ellipsenbogen um einen der Nebenscheitel der im Wesentlichen ellipsenförmigen Prothesenplatten 14, 16 entspricht. Wird der Anschlag 40 weitmöglichst in Richtung des distalen Endes des chirurgischen Instruments bewegt, wie in Figur 3a gezeigt, liegt die Anlagekontur 42 an der Außenkontur der Prothesenplatten 14, 16 an. Wird das chirurgische Instrument mit einem derartig eingestellten Anschlag 40 in den Zwischenwirbelraum eingeführt, schlägt die Anlagekontur 42 dann an den Zwischenwirbel an, sobald die Außenkontur der Prothesenplatten 14, 16 mit der Außenfläche der Wirbel übereinstimmt.

Zusätzlich weist der Anschlag 40 eine Markierung auf, welche mit einer fest am Griffelement 20 angeordneten Markierung in Übereinstimmung gebracht werden kann. Die Markierung gibt den Abstand von der Anlagekontur 42 zu dem Nebenscheitel der Prothesenplatten 14, 16 an, welcher das distale Ende des chirurgischen Instruments bildet. Vorliegend beträgt der Abstand 30 mm.

Figur 3b zeigt den weitmöglichst vom distalen Ende des chirurgischen Instruments zurückbewegten Anschlag 40, bei welcher die Markierung einen deutlich größeren Wert, nämlich 45 mm, anzeigt.

In Figur 3c ist eine Ansicht von oben axial auf das zweite Ausführungsbeispiel gezeigt, in welcher insbesondere die parallel verlaufenden Führungsschienen 34 zur Aufnahme der Prothesenplatten 14, 16 deutlich zu erkennen sind. In den Zwischenraum zwischen den Führungsschienen 34 wird der Prothesenkern 12 positioniert und durch Zusammenführen der beiden Halterungselemente 50, 52 fixiert gehalten.

In einem Operationsverfahren wird das erfindungsgemäße chirurgische Instrument auf folgender Weise verwendet. Nach dem der Zugang zur Wirbelsäule freigelegt und der Zwischenwirbelraum von der erkrankten Bandscheibe befreit und ausgeräumt wurde, wird zunächst mit einer Kontrollprothese die Größe des Zwischenraumes sondiert. Dabei wird einerseits die Größe der zu verwendenden Zwischenwirbelprothese, d. h. die Länge der Hauptachse der im Wesentlichen ellipsenförmigen Prothesenplatten 14, 16 und die Dicke der zu verwendenden Zwischenwirbelprothese 10, d.h. insbesondere die Dicke des Prothesenkerns 12, bestimmt. Des Weiteren wird die Neigung der Wirbelflächen zueinander bestimmt. Daraus ergibt sich, ob Prothesenplatten mit einer ebenen oder einer geneigten Rückenfläche verwendet werden.

Mit der Kontrollprothese wird ebenfalls bestimmt, wieweit die Zwischenwirbelprothese 10 in den Zwischenwirbelraum eingeführt werden muss. Der Abstand zwischen Außenkontur der Wirbel und gewünschter Position der Außenkontur der Zwischenwirbelprothese 10 wird an dem Anschlag 40 entsprechend eingestellt.

Die Zwischenwirbelprothese 10 wird anschließend aus den gewählten Bestandteilen, nämlich dem gewünschten Prothesenkern 12 und den optimierten Prothesenplatten 14, 16 zusammengesetzt und in das zweite Ausführungsbeispiel des erfindungsgemäßen chirurgischen Instruments gemäß Figuren 3a und 3b eingespannt. Der Prothesenkern 12 wird dabei von den Blattfedern 32 gehalten, während die Prothesenplatten 14, 16 in die Führungsschienen 34 eingesetzt und über das Zusammenspiel der Kugelsegmente 12a des Prothesenkerns 12 und den kugelschalenförmigen Vertiefungen 14b,16b der Prothesenplatten 14, 16 gehalten werden. Um die Zwischenwirbelprothese 10 einsetzen zu können, muss der Zwischenwirbelraum mit einem weiteren Spreizinstrument derart aufgeweitet werden, dass die Zwischenwirbelprothese 10 einschließlich der hervorstehenden Zähne 18 in den Zwischenwirbelraum eingeführt werden kann. Dabei ist das Aufspreizinstrument derart ausgebildet, dass das chirurgische Instrument zum Einsetzen von Zwischenwirbelprothesen gemäß der Erfindung problemlos in den Zwischenwirbelraum eingefügt werden kann, ohne dass die Valven des Aufspreizinstruments stören.

Das chirurgische Instrument mit der eingesetzten Zwischenwirbelprothese 10 wird nun in den Zwischenwirbelraum eingeführt, bis der Anschlag 40 auf den Wirbel auftrifft. Die Aufspreizelemente werden nun abgesenkt, bis die Wirbel in Eingriff mit den Zähnen 18 stehen. Anschließend kann das chirurgische Instrument gefahrlos entfernt werden, ohne die Position der Zwischenwirbelprothese 10 zu verändern, da diese über die Zähne 18 an den Wirbeln gehalten wird.

Der Vorteil dieser Operationsmethode liegt darin, dass eine geringere Aufspreizung des Zwischenwirbelraums nötig ist, da ein Einbringen des Prothesenkerns 12 zwischen die Prothesenplatten 14, 16, wobei die Kugelsegmente 12a über die Ringwülste 14a, 16a in die kugelschalenförmigen Vertiefungen 14b, 16b gepresst werden müssen, entfällt. Des Weiteren ist eine exakte Positionierung der Prothesenplatten 14, 16 und des Prothesenkerns 12 zueinander automatisch gewährleistet. Insbesondere wird auch eine Beschädigung der Oberfläche des Prothesenkerns 12 vermieden, da der Prothesenkern 12 gleichzeitig mit den Prothesenplatten 14, 16 in den Zwischenwirbelraum eingesetzt und nicht nachträglich in den Zwischenraum zwischen den beiden Prothesenplatten 14, 16 eingeschlagen wird.

### Bezugszeichenliste

- 10: Zwischenwirbelprothese
- 12: Prothesenkern
- 12a: Kugelsegment
- 12b: Ringsteg
- 13: Prothesenkern
- 13a: Mittelelement
- 13b: kugelschalenfömrige Vertiefung
- 13c: Gleitelement
- 14: Prothesenplatte
- 14a: Ringwulst
- 14b: kugelschalenförmige Vertiefung
- 16: Prothesenplatte
- 16a: Ringwulst
- 16b: kugelschalenförmige Vertiefung
- 18: Zähne

- 20: Griffelement
- 22: radiale Öffnung
- 24: Gewinde
- 26: Schraubelement

- 30: U-förmige Halterung
- 30a: Schenkel
- 30b: Schenkel
- 32: Blattfeder
- 34: Führungsschiene
- 36: Innenkontur

- 40: Anschlag
- 42: Anlagekontur

- 50: Halterungselement
- 52: Halterungselement

- 60: Parallelführung
- 60a: Scherenglied
- 60b: Scherenglied
- 61: freies Ende
- 62: freies Ende
- 63a: Verbindungselement
- 63b: Verbindungselement
- 65: Achse
- 66: Drehknopf

## Patentansprüche

1. Chirurgisches Instrument zum Einführen einer Zwischenwirbelprothese (10) bestehend aus wenigstens drei Bestandteilen, nämlich zwei Prothesenplatten (14, 16) und einem Prothesenkern (12), welches am distalen Ende eines Griffelements (20) eine U-förmige Halterung (30) aufweist, in welcher die Zwischenwirbelprothese (10) mit sämtlichen Bestandteilen lösbar einsetzbar ist, wobei die einzelnen Bestandteile zueinander fixiert ausgerichtet sind, **dadurch gekennzeichnet, dass** im Anschluss an die U-förmige Halterung (30) ein einziger entlang der Achse des Griffelements (20) bewegbarer Anschlag (40) angeordnet ist.

2. Chirurgisches Instrument nach Anspruch 1,
**dadurch gekennzeichnet, dass** die U-förmige Halterung (30) Klemmelemente für die Zwischenwirbelprothese (10) aufweist.

3. Chirurgisches Instrument nach Anspruch 2,
**dadurch gekennzeichnet, dass** die Klemmelemente als zwei auf der Innenseite entlang der Schenkel (30a, 30b) der U-förmigen Halterung (30) verlaufende Blattfedern (32) ausgebildet sind.

4. Chirurgisches Instrument nach einem der Ansprüche 2 oder 3,
**dadurch gekennzeichnet, dass** die Klemmelemente den Prothesenkern (12) halten.

5. Chirurgisches Instrument nach einem der vorhergehenden Ansprüche,
**dadurch gekennzeichnet, dass** die Innenkontur (36) der U-förmigen Halterung (30) korrespondierend zu der Außenkontur des Prothesenkerns (12) ausgebildet ist.

6. Chirurgisches Instrument nach Anspruch 5,
**dadurch gekennzeichnet, dass** die Innenkontur (36) der U-förmigen Halterung (30) als Kreisbogen von etwa 200° ausgebildet ist.

7. Chirurgisches Instrument nach einem der vorhergehenden Ansprüche,
**dadurch gekennzeichnet, dass** in der Ebene der U-förmigen Halterung (30) auf der Innenseite der Schenkel (30a, 30b) der U-förmigen Halterung (30) je zwei Führungsschienen (34) zur Aufnahme der Prothesenplatten (14, 16) ausgeformt sind.

8. Chirurgisches Instrument nach Anspruch 7,
**dadurch gekennzeichnet, dass** die Führungsschienen (34) parallel zueinander verlaufen.

9. Chirurgisches Instrument nach Anspruch 7 oder 8,
**dadurch gekennzeichnet, dass** die Führungsschienen (34) beidseitig der entlang der Schenkel (30a, 30b) der U-förmigen Halterung (30) verlaufenden Blattfedern (32) angeordnet sind.

10. Chirurgisches Instrument nach einem der vorhergehenden Ansprüche,
**dadurch gekennzeichnet, dass** der Anschlag (40) senkrecht zur Ebene der U-förmigen Halterung (30) angeordnet ist.

11. Chirurgisches Instrument nach einem der vorhergehenden Ansprüche,
**dadurch gekennzeichnet, dass** der Anschlag (40) das Griffelement (20) senkrecht durchsetzt und beidseitig des Griffelements (20) hervorragt.

12. Chirurgisches Instrument nach einem der vorhergehenden Ansprüche,
**dadurch gekennzeichnet, dass** in den Anschlag (40) ein Gewinde (24) eingreift, welches entlang der Achse des Griffelements (20) verläuft und mittels eines Schraubelements (26) verstellbar ist.

13. Chirurgisches Instrument nach einem der vorhergehenden Ansprüche,
**dadurch gekennzeichnet, dass** die U-förmige Halterung (30) zweiteilig ausgebildet ist, wobei die beiden Halterungselemente (50, 52) der U-förmigen Halterung (30) über eine Parallelführung (60) verbunden und aufspreizbar sowie schließbar sind.

14. Chirurgisches Instrument nach Anspruch 13,
**dadurch gekennzeichnet, dass** die beiden Halterungselemente (50, 52) der U-förmigen Halterung (30) an ihrer einander abgewandten Seite auf der Innenseite der Schenkel (30a, 30b) je eine Führungsschiene (34) zur Aufnahme der Prothesenplatten (14, 16) aufweisen.

## Claims

1. Surgical instrument for inserting an intervertebral prosthesis (10) consisting of at least three components, namely two prosthesis plates (14, 16) and a prosthesis core (12), which at the distal end of a gripping element (20) has a U-shaped holder (30), in which the intervertebral prosthesis (10) with all its components can be inserted detachably, wherein the individual components are oriented fixed relative to one another, **characterized in that** a single stop (40) is arranged movably along the axis of the gripping element (20) and connected to the U-shaped holder (30).

2. Surgical instrument according to Claim 1, **characterized in that** the U-shaped holder (30) has clamping elements for the intervertebral prosthesis (10).

3. Surgical instrument according to Claim 2, **characterized in that** the clamping elements are formed as two leaf springs (32) extending on the inside along the legs (30a, 30b) of the U-shaped holder (30).

4. Surgical instrument according to one of Claims 2 or 3, **characterized in that** the clamping elements hold the prosthesis core (12).

5. Surgical instrument according to one of the preceding claims, **characterized in that** the internal contour (36) of the U-shaped holder (30) is formed corresponding to the external contour of the prosthesis core (12).

6. Surgical instrument according to Claim 5, **characterized in that** the internal contour (36) of the U-shaped holder (30) is formed as a circular arc of about 200°.

7. Surgical instrument according to one of the preceding claims, **characterized in that** in each case two guide rails (34) for receiving the prosthesis plates (14, 16) are formed in the plane of the U-shaped holder (30), on the inside of the legs (30a, 30b) of the U-shaped holder (30).

8. Surgical instrument according to Claim 7, **characterized in that** the guide rails (34) run parallel to one another.

9. Surgical instrument according to Claim 7 or 8, **characterized in that** the guide rails (34) are arranged either side of the leaf springs (32) extending along the legs (30a, 30b) of the U-shaped holder (30).

10. Surgical instrument according to one of the preceding claims, **characterized in that** the stop (40) is arranged perpendicularly to the plane of the U-shaped holder (30).

11. Surgical instrument according to one of the preceding claims, **characterized in that** the stop (40) passes through the gripping element (20) perpendicularly and projects on either side of the gripping element (20).

12. Surgical instrument according to one of the preceding claims, **characterized in that** a thread (24), which extends along the axis of the gripping element (20) and is adjustable by means of a screw element (26), engages in the stop (40).

13. Surgical instrument according to one of the preceding claims, **characterized in that** the U-shaped holder (30) is formed in two parts, wherein the two holding elements (50, 52) of the U-shaped holder (30) are connected by a parallel guide (60) and can be opened and closed.

14. Surgical instrument according to Claim 13, **characterized in that** the two holding elements (50, 52) of the U-shaped holder (30) have, on the side opposite to one another on the inside of the legs (30a, 30b), in each case a guide rail (34) for receiving the prosthesis plates (14, 16).

## Revendications

1. Instrument chirurgical pour l'introduction d'un implant intervertébral (10) constitué d'au moins trois éléments, à savoir deux plaques d'implant (14, 16) et un noyau d'implant (12), comportant à l'extrémité distale d'un élément de préhension (20) un support (30) en forme de U dans lequel l'implant intervertébral (10) peut être logé de façon amovible avec la totalité de ces éléments, ces éléments étant orienté en étant fixés les uns aux autres,
**caractérisé en ce qu'**
une butée unique (40) mobile le long de l'axe de l'élément de préhension (20) est reliée au support en forme de U (30).

2. Instrument chirurgical conforme à la revendication 1,
**caractérisé en ce que**
le support (30) en forme de U comporte des éléments de blocage de l'implant intervertébral (10).

3. Instrument chirurgical conforme à la revendication 2,
**caractérisé en ce que**
les éléments de blocage sont réalisés sous la forme de deux ressorts à lames (32) s'étendant, le long de la face interne des branches (30a, 30b) du support (30) en forme de U.

4. Instrument chirurgical conforme à la revendication 2 ou 3,
**caractérisé en ce que**
les éléments de blocage maintiennent le noyau de l'implant (12).

5. Instrument chirurgical conforme à l'une des revendications précédentes,
**caractérisé en ce que**
la périphérie interne (36) du support (30) en forme de U est réalisée de façon à correspondre à la périphérie externe du noyau de l'implant (12).

6. Instrument chirurgical conforme à la revendication 5,
**caractérisé en ce que**
la périphérie interne (36) du support (30) en forme de U est réalisé sous la forme d'un arc de cercle d'environ 200°.

7. Instrument chirurgical conforme à l'une des revendications précédentes,
**caractérisé en ce que**
dans le plan du support (30) en forme de U, et sur la face interne des branches (30a, 30b) de ce support (30) sont respectivement formés deux rails de guidage (34) pour la réception des plaques d'implant (14, 16).

8. Instrument chirurgical conforme à la revendication 7,
**caractérisé en ce que**
les rails de guidage (34) s'étendent parallèlement l'un à l'autre.

9. Instrument chirurgical conforme à la revendication 7 ou 8,
**caractérisé en ce que**
les rails de guidage (34) sont disposés de part et d'autre des ressorts à lames (32) s'étendant le long des branches (30a, 30b) du support (30) en forme de U.

10. Instrument chirurgical conforme à l'une des revendications précédentes,
**caractérisé en ce que**
la butée (40) est disposée perpendiculairement au plan du support (30) en forme de U.

11. Instrument chirurgical conforme à l'une des revendications précédentes,
**caractérisé en ce que**
la butée (40) traverse perpendiculairement l'élément de préhension (20) et dépasse de part et d'autre de cet élément de préhension (20).

12. Instrument chirurgical conforme à l'une des revendications précédentes,
**caractérisé en ce que**
un filetage (24) qui s'étend le long de l'axe de l'élément de préhension (20) et peut être réglé au moyen d'un élément fileté (26) vient en prise dans la butée (40).

13. Instrument chirurgical conforme à l'une des revendications précédents,
**caractérisé en ce que**
le support (30) en forme de U est réalisé en deux parties, les deux éléments supports (50, 52) du support (30) en forme de U étant reliés par l'intermédiaire d'un parallélogramme articulé (60) et pouvant être écartés ou fermés.

14. Instrument chirurgical conforme à la revendication 13,
**caractérisé en ce que**
les deux éléments supports (50, 52) du support (30) en forme de U comportent, respectivement, sur leurs faces opposées, sur la face interne des branches (30a, 30b) un rail de guidage (34) pour la réception des plaques d'implants (1A,16).
